# EUROPÄISCHE PATENTSCHRIFT

(11) **EP 1 054 017 B1**
(45) Veröffentlichungstag und Bekanntmachung des Hinweises auf die Patenterteilung: **13.10.2004**
(21) Anmeldenummer: 00108622.2
(22) Anmeldetag: 20.04.2000
(51) Int. Cl.: C07K 5/065, A61K 38/05, A61P 7/02

(54) **Salze von Thrombininhibitoren**
Salts of thrombin inhibitors
Sels d'inhibiteurs de thrombine

(30) Priorität: 10.05.1999 DE 19921345; 01.10.1999 DE 19947479
(43) Veröffentlichungstag der Anmeldung: 22.11.2000
(73) Patentinhaber: Abbott GmbH & Co. KG, 65205 Wiesbaden (DE)
(72) Erfinder: Schäfer, Bernd, Dr., 76889 Dierbach (DE)
(74) Vertreter: Grünecker, Kinkeldey, Stockmair & Schwanhäusser Anwaltssozietät

(56) Entgegenhaltungen:
- WO-A-94/29336
- WO-A-96/25426

## Beschreibung

Die Erfindung betrifft neue Salze von Thrombininhibitoren, deren Herstellung und Verwendung zur Herstellung von Arzneimitteln mit antithrombotischer Wirkung.

Speziell betrifft die Erfindung neue Verbindungen der allgemeinen Formel I oder II 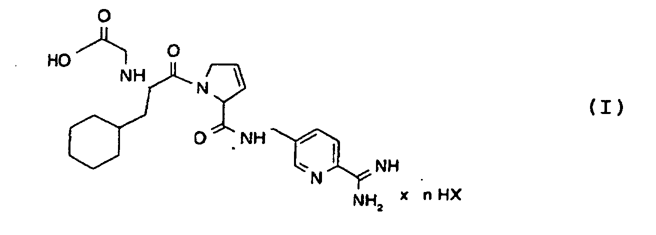 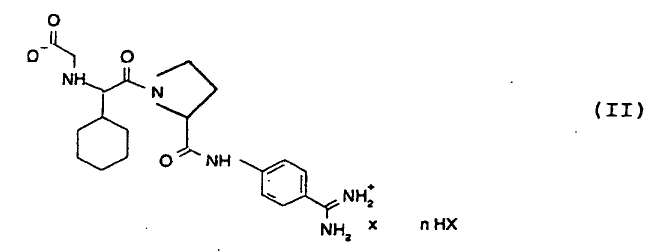 wobei
n 0.5, 1, 2 und
HX 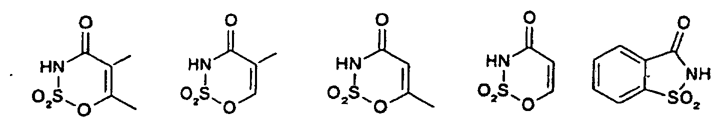 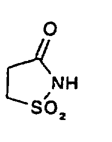 bedeutet und HX optional 1- oder 2-fach mit Methyl, Ethyl oder Hydroxy substituiert ist, sowie deren Tautomere und Stereoisomere entspricht.

Bevorzugt sind folgende Bedeutungen von HX:

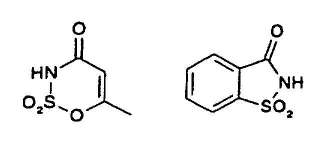

Besonders bevorzugt sind die R-Konfiguration des Cyclohexylalanins und die S-Konfiguration des Dehydroprolins des Wirkstoffs der allgemeinen Formel I.

Die Herstellung des Thrombininhibitors I mit HX = HOAc ist in WO 96/25426, die des Thrombininhibitors II mit n = 0 ist in WO 94/29336 beschrieben. Die Salze sind in der Regel amorph. Die Lagerung dieser Produkte, insbesondere bei erhöhter Temperatur, führt zur Bildung einer Reihe von Nebenprodukten.

Der Erfindung lag also die Aufgabe zugrunde, lagerstabile Wirkstoffformen zu finden.

Die Betaine der Wirkstoffe erhält man durch Titration der sauren Salze bis zum isoelektrischen Punkt, Fällung oder Kristallisation, Filtration oder Zentrifugation und Trocknung. Die Salze der allgemeinen Formel I und II erhält man, indem man die Betaine mit HX umsetzt oder wenn man das Alkalisalz von HX mit den Mineralsäuresalzen oder C₁-C₄-Carbonsäuresalzen von I oder II umsetzt. Als Lösungsmittel kommen Wasser, C₁-C₆-Alkohole, C₁-C₆-Ether, C₁-C₆-Ester, Toluol, Xylole, DMF, DMSO, THF in Frage. Ganz bevorzugtes Lösungsmittel ist Wasser. Die Isolierung des Produktes geschieht durch Filtration oder Zentrifugation und Trocknung oder aus einer Lösung durch Gefriertrocknung oder Sprühtrocknung.

Zur Erzeugung von Kristallen bieten sich alle gängigen Methoden wie z.B. beschrieben in Houben-Weyl, Band I/1, Georg Thieme Verlag, Stuttgart, 1958, 341; Römpps Chemie-Lexikon, 8. Auflage, Franckh'sche Verlagshandlung, Stuttgart, 1983, 2244, und der dort zitierten Literatur an.

In der Regel sind die üblichen physiologisch verträglichen Salze von I und II amorphe Feststoffe. Wir haben nun überraschenderweise gefunden, daß insbesondere die Salze mit Acesulfam-K und Saccharin gut kristallisierende Verbindungen sind. Sie fallen spontan aus Wasser aus. Aber auch die Zugabe minimaler Mengen an Wasser im Bereich von 5 bis 500 Gew.-% zu einer festen, stöchiometrischen Mischung von 1 oder 2 mit HX reichen aus, um kristalline Substanzen zu erhalten.

Die Salzbildung wird üblicherweise diskontinuierlich in Reaktionskesseln durchgeführt. Aber auch eine kontinuierliche Produktion, z.B. in einer Rührkesselkaskade oder in einem Extruder, ist möglich. Der Prozeß ist so robust, daß er auch durch Mischen von Feststoffen in dafür geeigneten Apparaten, bevorzugt in solchen für die galenische Verarbeitung von Pharmawirkstoffen, durchgeführt werden kann.

Die Kristallisationstemperatur liegt im allgemeinen im Bereich von -80 bis 200°C, bevorzugt im Bereich von -20 bis 150°C.

Der Druck liegt im Bereich von 1 bar bis 2000 bar.

Die Kristallinität der Proben wurde an Hand von Debye-Scherrer-Aufnahmen beurteilt.

In einem Stabilitätstest wurde die thermische Zersetzung der Verbindungen der allgemeinen Formel I und II untersucht. Hierzu wurden die Verbindungen I oder II 10 Tage bei 70°C bei Normaldruck an der Luft gelagert und im Abstand von jeweils 7 Tagen die relative Abnahme des Gehaltes durch HPLC-Analyse bestimmt.

Wir haben nun überraschend gefunden, daß die Salze der allgemeinen Formel I und II wesentlich stabiler sind als das Betain und die Salze von Mineralsäuren und C₁-C₄-Carbonsäuren. Besonders vorteilhaft für die Stabilität des Wirkstoffs ist die Kristallinität der Salze.

Die neuen Salze der allgemeinen Formel I oder II lassen sich bei folgenden Indikationen einsetzen:
- Krankheiten, deren Pathomechanismus direkt oder indirekt auf der proteolytischen Wirkung von Thrombin beruht,
- Krankheiten, deren Pathomechanismus auf der thrombinabhängigen Aktivierung von Rezeptoren und Signaltransduktionen beruht,
- Krankheiten, die mit Stimulation (z.B. durch PAI-1, PDGF (plated derived growth factor), P-Selectin, ICAM-1, Tissue Factor oder Inhibition (z.B. NO-Synthese in Glattmuskelzellen) von Genexpressionen in Körperzellen einhergehen,
- Krankheiten, die auf der mitogenen Wirkung von Thrombin beruhen,
- Krankheiten, die auf einer thrombinabhängigen Kontraktilitäts- und Permeabilitätsveränderung von Epithelzellen (z.B. Gefäßendothelzellen) beruhen,
- thrombinabhängige, thromboembolische Ereignisse wie tiefe Venenthrombose, Lungenembolie, Myocard- oder Cerebralinfarkt, Vorhofflimmern, Bypassverschluß,
- disseminierte intravasale Koagulation (DIC).,
- Reckklusion und zur Verkürzung der Reperfusionszeit bei Komedikation mit Thrombolytika wie Streptokinase, Urokinase, Prourokinase, t-PA, APSAC, Plasminogenaktivatoren aus den Speicheldrüsen von Tieren sowie die rekombinanten und mutierten Formen all dieser Substanzen,
- das Auftreten von früher Reokklusion und später Restenosierung nach PTCA,
- die thrombinabhängige Proliferation von Glattmuskelzellen,
- die Akkumulation aktiven Thrombins im ZNS (z.B. bei M. Alzheimer),
- das Tumorwachstum sowie gegen die Adhäsion und Metastasierung von Tumorzellen.

Insbesondere lassen sich die neuen Verbindungen zur Therapie und Prophylaxe von thrombinabhängigen thromboembolischen Ereignissen wie tiefen Venenthrombosen, Lungenembolien, Myocard- oder Cerebralinfarkten und instabiler Angina, weiterhin zur Therapie der Disseminierten Intravasalen Koagulation (DIC) einsetzen. Weiter eignen sie sich zur Kombinationstherapie mit Thrombolytika wie Streptokinase, Urokinase, Prourokinase, t-PA, APSAC und anderen Plasminogenaktivatoren zur Verkürzung der Reperfusionszeit und Verlängerung der Reokklusionszeit.

Weitere bevorzugte Anwendungsgebiete sind die Verhinderung thrombinabhängiger früher Reckklusion und später Restenosierung nach perkutaner transluminaler koronarer Angioplastie, die Verhinderung thrombininduzierter Proliferation glatter Muskelzellen, die Verhinderung der Akkumulation aktiven Thrombins im ZNS (z.B. bei M. Alzheimer), die Tumorbekämpfung und die Verhinderung von Mechanismen, die zu Adhäsion und Metastasierung von Tumorzellen führen.

Die neuen Verbindungen lassen sich auch zur Beschichtung von künstlichen Oberflächen wie Hämodialysemembranen und den dazu erforderlichen Schlauchsystemen und Leitungen sowie von Oxagenatoren der extraversalen Zirkulation, Stents und Herzklappen verwenden.

Die neuen Verbindungen lassen sich weiter bei Krankheiten einsetzen, deren Pathomechanismus direkt oder indirekt auf der proteolytischen Wirkung von Kininogenasen, insbesondere Kallikrein, beruht, z.B. bei Entzündungskrankheiten wie Asthma, Pankreatitis, Rhinitis, Arthritis, Urticaria und anderen inneren Entzündungskrankheiten.

Die erfindungsgemäßen Verbindungen können in üblicher Weise oral oder parenteral (subkutan, intravenös, intramuskulär, intraperitoneal, rektal) verabfolgt werden. Die Applikation kann auch mit Dämpfen oder Sprays durch den Nasen-Rachenraum erfolgen. Insbesondere können die Verbindungen oral gegeben werden.

Die Dosierung hängt vom Alter, Zustand und Gewicht des Patienten sowie von der Applikationsart ab. In der Regel beträgt die tägliche Wirkstoffdosis pro Person zwischen etwa 10 und 2000 mg bei oraler Gabe und zwischen etwa 1 und 200 mg bei parenteraler Gabe. Diese Dosis kann in 2 bis 4 Einzeldosen oder einmalig am Tag als Depotform gegeben werden.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al.: Pharmazeutische Technologie, Thieme-Verlag, Stuttgart, 1978). Die so erhaltenen Applikationsformen enthalten den Wirkstoff normalerweise in einer Menge von 0,1 bis 99 Gew.-%.

### Vergleichsbeispiele

### 1. Beispiel

### N-((Hydroxycarbonyl)-methylen)-(R)-cyclohexylalanyl-(S)-3,4-dehydroprolin-(6-amidino-3-picolinyl)-amid (Betain)

90 g (0,116 mol) der in WO 98/09950 Beispiel 3 beschriebenen Verbindung N-Boc-N-((t-Butoxycarboriyl)-methylen)-(R)-cyclohexylalanyl-(S)-3,4-dehydroprolin-(6-amidino-3-picolinyl)-amid-N-acetyl-(S)-cysteinat, 360 g Wasser und 44,56 g (38 %ig, 0,464 mol) Salzsäure wurden 2 h auf 65°C erwärmt. Nach dem Abkühlen auf Raumtemperatur wurde 1 mal mit Essigester extrahiert, die Phasen getrennt und mit 105 g einer 25 %igen wäßrigen Ammoniaklösung pH 8,2 eingestellt. Hierbei fiel das Produkt aus. Man rührte noch eine Stunde nach, saugte ab, wusch mit Eiswasser und trocknete anschließend im Vakuumtrockenschrank. Es wurden 41,66 g (0,091 mol, 79 %) N-((Hydroxycarbonyl)-methylen)-(R)-cyclohexylalanyl-(S)-3,4-dehydroprolin-(6-amidino-3-picolinyl)-amid in Form eines farblosen Feststoffs erhalten. Debye-Scherrer-Aufnahme: amorph

### 2. Beispiel

### N-((Hydroxycarbonyl)-methylen)-(R)-cyclohexylalanyl-(S)-3,4 -dehydroprolin-(6-amidino-3-picolinyl)-amid-fumarat

3 g (6,6 mmol) N-((Hydroxycarbonyl)-methylen)-(R)-cyclohexylalanyl-(S)-3,4-dehydroprolin-(6-amidino-3-picolinyl)-amid wurden in 30 ml Wasser vorgelegt und 0,686 g (6 mmol) Fumarsäure zugegeben. Der Reaktionsansatz wurde über Nacht bei Raumtemperatur gerührt und anschließend gefriergetrocknet. Es wurden 3,2 g (5,6 mmol, 93 %) N-((Hydroxycarbonyl)-methylen)-(R)-cyclohexylalanyl-(S)-3,4-dehydroprolin-(6-amidino-3-picolinyl)-amidfumarat in Form eines farblosen Pulvers erhalten.
Debye-Scherrer-Aufnahme: amorph

### 3. Beispiel

### N-((Hydroxycarbonyl)-methylen)-(R)-cyclohexylalanyl-(S)-3,4-dehydroprolin-(6-amidino-3-picolinyl)-amid-acetat

3 g (6,6 mmol) N-((Hydroxycarbonyl)-methylen)-(R)-cyclohexylalanyl-(S)-3,4-dehydroprolin-(6-amidino-3-picolinyl)-amid wurden in 30 ml Wasser vorgelegt und 0,39 g (6,5 mmol) Essigsäure zugegeben. Der Reaktionsansatz wurde über Nacht bei Raumtemperatur gerührt und anschließend gefriergetrocknet. Es wurden 3,4 g (6,5 mmol, 100 %) N-((Hydroxycarbonyl)-methylen)-(R)-cyclohexylalanyl-(S)-3,4-dehydroprolin-(6-amidino-3-picolinyl)-amidacetat in Form eines farblosen Pulvers erhalten.
Debye-Scherrer-Aufnahme: amorph

### Beispiele

### 4. Beispiel

### N-((Hydroxycarbonyl)-methylen)-(R)-cyclohexylalanyl-(S)-3,4-dehydroprolin-(6-amidino-3-picolinyl)-amid-acesulfamat

3,5 g (6,6 mmol) N-((Hydroxycarbonyl)-methylen)-(R)-cyclohexylalanyl-(S)-3,4-dehydroprolin-(6-amidino-3-picolinyl)-amid-hydrochlorid in 15 ml Wasser und 1,33 g (6,6 mmol) Acesulfam-K in 15 ml Wasser wurden zusammengegeben. Nach ca. 2,5 Stunden begann das Produkt auszukristallisieren. Nach Rühren über Nacht wurde das Produkt abfiltriert, einmal mit 5 ml Wasser gewaschen und im Trockenschrank bei 70°C getrocknet. Es wurden 1,4 g (2,4 mmol, 37 %) N-((Hydroxycarbonyl)-methylen)-(R)-cyclohexylalanyl-(S)-3,4-dehydroprolin-(6-amidino-3-picolinyl)-amid-acesulfamat in Form eines farblosen Pulvers erhalten.
Debye-Scherrer-Aufnahme: kristallin

### 5. Beispiel

### N-((Hydroxycarbonyl)-methylen)-(R)-cyclohexylalanyl-(S)-3,4-dehydroprolin-(6-amidino-3-picolinyl)-amid-saccharinat

3 g (6,6 mmol) N-((Hydroxycarbonyl)-methylen)-(R)-cyclohexylalanyl-(S)-3,4-dehydroprolin-(6-amidino-3-picolinyl)-amid wurden in 30 ml Wasser vorgelegt und 1,2 g (6,6 mmol) Saccharin zugegeben. Nach 1,5 h saugte man ab, wusch mit Wasser und trocknete im Vakuumtrockenschrank. Es wurden 2,5 g (3,9 mmol, 59 %) N-((Hydroxycarbonyl)-methylen)-(R)-cyclohexylalanyl-(S)-3,4-dehydroprolin-(6-amidino-3-picolinyl)-amid-saccharinat in Form eines farblosen Pulvers erhalten.
Debye-Scherrer-Aufnahme: kristallin

### 6. Beispiel

### N-((Hydroxycarbonyl)-methylen)-(R)-cyclohexylalanyl-(S)-3,4-dehydroprolin-(6-amidino-3-picolinyl)-amid-saccharinat

2,9 g (12 mmol) Saccharin-Natriumsalz wurden in 10 ml Wasser gelöst vorgelegt und 6,36 g (93 %ig, 12 mmol) N-((Hydroxycarbonyl)-methylen)-(R)-cyclohexylalanyl-(S)-3,4-dehydroprolin-(6-amidino-3-picolinyl)-amid-hydrochlorid in 30 ml Wasser gelöst, langsam zugetropft. Man ließ den Ansatz 4 h rühren, saugte ab, wusch mit Wasser nach und trocknete im Vakuumtrockenschrank. Es wurden 6 g (9,4 mmol, 78 %) N-((Hydroxycarbonyl)-methylen)-(R)-cyclohexylalanyl-(S)-3,4-dehydroprolin-(6-amidino-3-picolinyl)-amid-saccharinat in Form eines farblosen Pulvers erhalten.
Debye-Scherrer-Aufnahme: kristallin

### 7. Beispiel

### Stabilitätstests

Die Substanzen wurden bei 70°C, 1 bar gelagert. Gemessen wurde in HPLC Flächen-%

**Tabelle 1**

| Lagerstabilität von Salzen der Verbindung I im offenen Gefäß | | | | |
|---|---|---|---|---|
| Tage | Acesulfam-Salz n = 1 | Saccharin Salz n = 1 | Acetat n = 1 | Fumarat n = 1 |
| 0 | 100 | 100 | 100 | 100 |
| 7 | 96,7 | 100 | 92,6 | 87,9 |
| 14 | 88,4 | 101 | 87,2 | 87,1 |

**Tabelle 2**

| Lagerstabilität der Verbindung II im geschlossenen Gefäß | | |
|---|---|---|
| Tage | HCl-Salz | Saccharin-Salz |
| 0 | 100 | 100 |
| 7 | 99,3 | 100 |
| 14 | 98,9 | 100 |

## Patentansprüche

1. Verbindung der allgemeinen Formel I oder II 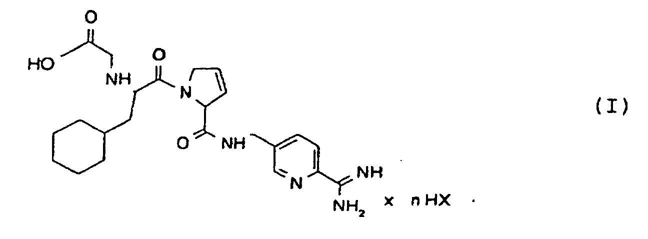 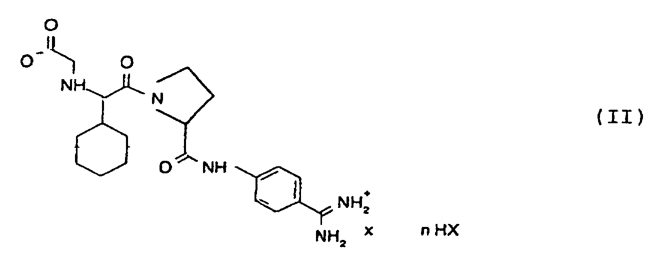 wobei
n 0.5, 1, 2 und
HX 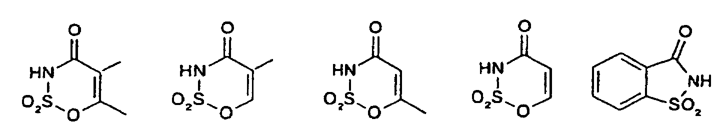 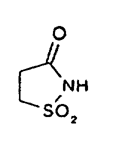 bedeutet und HX optional 1- oder 2-fach mit Methyl, Ethyl oder Hydroxy substituiert ist, sowie deren Tautomere und Stereoisomere bedeutet.

2. Verbindung nach Anspruch 1 in kristalliner Form.

3. Verfahren zur Herstellung der Verbindungen der allgemeinen Formel I nach einem der Ansprüche 1 oder 2, wobei das Betain von I oder II (n = 0) mit n HX umgesetzt wird.

4. Verfahren zur Herstellung der Verbindungen der allgemeinen Formel I nach einem der Ansprüche 1 oder 2, wobei ein Mineralsäuresalz oder C₁-C₄-Carbonsäuresalz des Wirkstoffs der Formel I oder II mit einem Alkalisalz von HX umgesetzt wird.

5. Verfahren nach Anspruch 3, **dadurch gekennzeichnet, daß** man die Verbindungen der allgemeinen Formel I oder II in Wasser herstellt.

6. Verfahren nach Anspruch 4, **dadurch gekennzeichnet, daß** man die Verbindungen der allgemeinen Formel I oder II in Wasser herstellt.

7. Verfahren nach Anspruch 6, **dadurch gekennzeichnet, daß** man in 5 bis 500 Gew.-% Wasser eine feste, stöchiometrische Mischung eines Mineralsäure- oder C₁-C₄-Carbonsäuresalzes von Verbindungen der allgemeinen Formel I oder II mit einem Alkalisalz von HX verwendet.

8. Arzneimittel, enthaltend Verbindungen der Formel I oder II gemäß einem der Ansprüche 1 oder 2 neben üblichen Träger- und Hilfsstoffen.

9. Verwendung der Verbindungen der Formel I oder II nach einem der Ansprüche 1 oder 2 zur Herstellung von Arzneimitteln zur Behandlung oder Prophylaxe von:
• Krankheiten, deren Pathomechanismus direkt oder indirekt auf der proteolytischen Wirkung von Thrombin beruht,
• Krankheiten, deren Pathomechanismus auf der thrombinabhängigen Aktivierung von Rezeptoren und Signaltransduktionen beruht,
• Krankheiten, die mit Stimulation oder Inhibition von Genexpressionen in Körperzellen einhergehen,
• Krankheiten, die auf der mitogenen Wirkung von Thrombin beruhen,
• Krankheiten, die auf einer thrombinabhängigen Kontraktilitäts- und Permeabilitätsveränderung von Epithelzellen beruhen,
• thrombinabhängige, thromboembolische Ereignisse,
• disseminierte intravasale Koagulation,
• Reokklusion und zur Verkürzung der Reperfusionszeit bei Komedikation mit Thrombolytika,
• das Auftreten von früher Reokklusion und später Restenosierung nach PTCA,
• die thrombinabhängige Proliferation von Glattmuskelzellen,
• die Akkumulation aktiven Thrombins im ZNS,
• das Tumorwachstum sowie gegen die Adhäsion und Metastasierung von Tumorzellen.

10. Verbindungen der Formel I oder II gemäß Anspruch 1, zur Beschichtung von Oberflächen.

## Claims

1. Compound of the general formula I or II 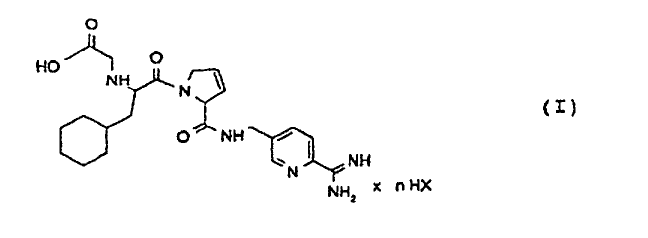 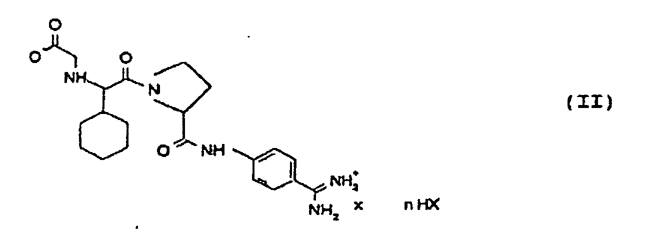 wherein
n represents 0.5, 1, 2 and
HX represents 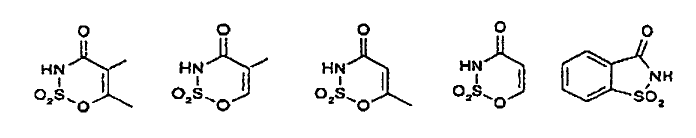 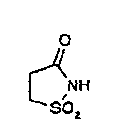 and HX is optionally substituted once or twice by methyl, ethyl or hydroxy, and their tautomers and stereoisomers.

2. Compound according to claim 1 in crystalline form.

3. Process for the preparation of compounds of the general formula I according to either claim 1 or claim 2, wherein the betaine of I or II (n = 0) is reacted with n HX.

4. Process for the preparation of compounds of the general formula I according to either claim 1 or claim 2, wherein a mineral acid salt or C₁-C₄-carboxylic acid salt of the active ingredient of formula I or II is reacted with an alkali metal salt of HX.

5. Process according to claim 3, **characterised in that** the compounds of the general formula I or II are prepared in water.

6. Process according to claim 4, **characterised in that** the compounds of the general formula I or II are prepared in water.

7. Process according to claim 6, **characterised in that** a solid, stoichiometric mixture of a mineral acid salt or C₁-C₄-carboxylic acid salt of compounds of the general formula I or II with an alkali metal salt of HX in from 5 to 500 wt.% water is used.

8. Medicament comprising compounds of formula I or II according to either claim 1 or claim 2, together with conventional carriers and auxiliary substances.

9. Use of compounds of formula I or II according to either claim 1 or claim 2 in the preparation of medicaments for the treatment or prophylaxis of:
• diseases whose pathogenetic mechanism is based directly or indirectly on the proteolytic action of thrombin,
• diseases whose pathogenetic mechanism is based on the thrombin-dependent activation of receptors and signal transductions,
• diseases which accompany stimulation or inhibition of gene expressions in body cells,
• diseases based on the mitogenic action of thrombin,
• diseases based on a thrombin-dependent change in the contractility and permeability of epithelial cells,
• thrombin-dependent thromboembolic events,
• disseminated intravascular coagulation,
• reocclusion and for shortening the reperfusion time on co-medication with thrombolytics,
• the occurrence of early reocclusion and late restenosis after PTCA,
• the thrombin-dependent proliferation of smooth muscle cells,
• the accumulation of active thrombin in the CNS,
• tumour growth and against the adhesion and metastasis of tumour cells.

10. Compounds of formula I or II according to claim 1, for the coating of surfaces.

## Revendications

1. Composé répondant à la formule générale I ou II 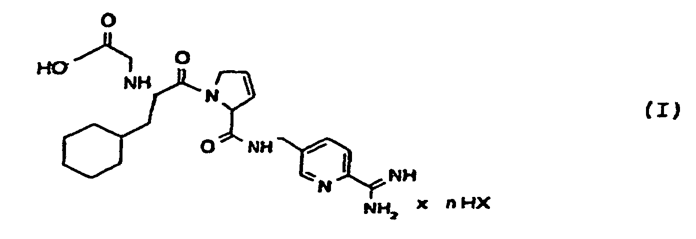 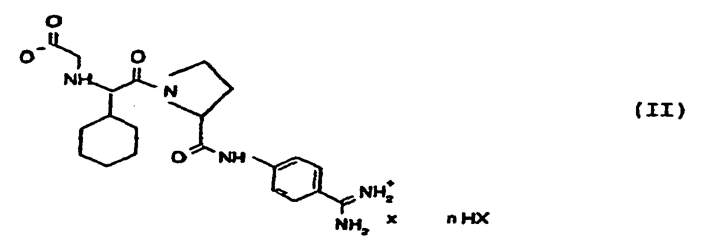 dans laquelle
n vaut 0,5, 1, 2 et
HX représente 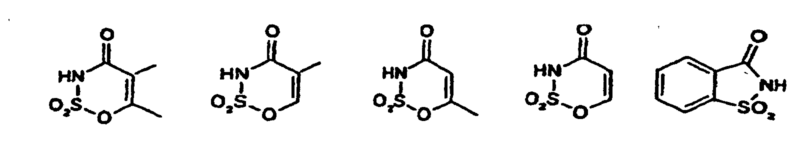 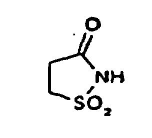 et HX est substitué facultativement 1 ou 2 fois par un groupe méthyle, un groupe éthyle ou un groupe hydroxy, et représente également leurs tautomères et stéréoisomères.

2. Composé selon la revendication 1 sous forme cristalline.

3. Procédé de production de composés répondant à la formule générale I selon l'une des revendications 1 ou 2, dans lequel la bétaïne de I ou II (n = 0) est mise en réaction avec n HX.

4. Procédé de production de composés répondant à la formule générale I selon l'une des revendications 1 ou 2, dans lequel un sel d'acide minéral ou un sel d'acide carboxylique en C1-C4 de la substance active de la formule I ou II est mis en réaction avec un sel alcalin de HX.

5. Procédé selon la revendication 3, **caractérisé en ce que** l'on produit dans l'eau les composés répondant à la formule générale I ou II.

6. Procédé selon la revendication 4, **caractérisé en ce que** l'on produit dans l'eau les composés répondant à la formule générale I ou II.

7. Procédé selon la revendication 6, **caractérisé en ce que** l'on utilise, dans 5 à 500 % en poids d'eau, un mélange stoechiométrique solide d'un sel d'acide minéral ou d'acide carboxylique en C1-C4 de composés répondant à la formule générale I ou II avec un sel alcalin de HX.

8. Médicaments contenant des composés répondant à la formule I ou II selon l'une des revendications 1 ou 2, en plus des excipients et adjuvants habituels.

9. Utilisation des composés répondant à la formule I ou II selon l'une des revendications 1 ou 2 pour la production de médicaments pour le traitement ou la prophylaxie de :
• maladies dont le pathomécanisme repose directement ou indirectement sur l'action protéolytique de la thrombine,
• maladies dont le pathomécanisme repose sur l'activation, en fonction de la thrombine, de récepteurs et de transductions de signaux,
• maladies qui sont liées à la stimulation ou à l'inhibition d'expressions géniques dans les cellules corporelles,
• maladies qui reposent sur le pouvoir mitogène de la thrombine,
• maladies qui reposent sur une altération de la contractilité et de la perméabilité des cellules épithéliales en fonction de la thrombine,
• phénomènes thromboemboliques en fonction de la thrombine,
• la coagulation intravasculaire disséminée,
• la réocclusion, et pour la réduction du temps de reperfusion en cas d'association médicamenteuse avec des thrombolytiques,
• l'apparition de réocclusion précoce et de resténose tardive après angioplastie percutanée coronaire transluminale,
• la prolifération, en fonction de la thrombine, de cellules des muscles lisses,
• l'accumulation de thrombine active dans le système nerveux central,
• la croissance tumorale, ainsi que l'adhérence et la dissémination de cellules tumorales.

10. Composés répondant à la formule I ou II selon la revendication 1, pour le revêtement de surfaces.
